# FASCICULE DE BREVET EUROPEEN

(11) **EP 3 186 357 B1**
(45) Date de publication et mention de la délivrance du brevet: **04.09.2019**
(21) Numéro de dépôt: 15763059.1
(22) Date de dépôt: 26.08.2015
(51) Int. Cl.: C12N 1/12, C12P 7/64

(54) **NOUVEAU PROCEDE DE CULTURE DE MICROALGUES**
NEUARTIGES VERFAHREN ZUR ZÜCHTUNG VON MIKROALGEN
NOVEL METHOD FOR CULTURE OF MICROALGAE

(30) Priorité: 27.08.2014 FR 1458025
(43) Date de publication de la demande: 05.07.2017
(73) Titulaire: Fermentalg, 33500 Libourne (FR)
(72) Inventeur: CALLEJA, Pierre, 33500 Libourne (FR); ROLS, Cyril, F-24000 Perigeux (FR); PAGLIARDINI, Julien, F-33300 Bordeaux (FR); BOURDENX, Brice, F-33700 Merignac (FR)
(74) Mandataire: Be IP
(86) Numéro de dépôt international: PCT/FR2015/052274
(87) Numéro de publication internationale: WO 2016/030631

(56) Documents cités:
- EP-A1- 0 657 543
- EP-A1- 2 194 138
- WO-A1-2005/035775
- WO-A2-2014/074770
- ES-A1- 2 333 571
- JP-A- H0 965 871
- MARTIN E. DE SWAAF ET AL: "High-cell-density fed-batch cultivation of the docosahexaenoic acid producing marine algaCrypthecodinium cohnii", BIOTECHNOLOGY AND BIOENGINEERING, vol. 81, no. 6, 20 mars 2003 (2003-03-20), pages 666-672, XP055114173, ISSN: 0006-3592, DOI: 10.1002/bit.10513
- JIANG Y ET AL: "EFFECTS OF SALINITY ON CELL GROWTH AND DOCOSAHEXAENOIC ACID CONTENT OF THE HETEROTROPHIC MARINE MICROALGA CRYPTHECODINIUM COHNII", JOURNAL OF INDUSTRIAL MICROBIOLOGY AND BIOTECHNOLOGY, BASINGSTOKE, GB, vol. 23, no. 6, 1999, pages 508-513, XP008042870, ISSN: 1367-5435, DOI: 10.1038/SJ.JIM.2900759
- ANA MENDES ET AL: "Crypthecodinium cohnii with emphasis on DHA production: a review", JOURNAL OF APPLIED PHYCOLOGY, KLUWER ACADEMIC PUBLISHERS, DO, vol. 21, no. 2, 17 août 2008 (2008-08-17), pages 199-214, XP019678371, ISSN: 1573-5176
- FABIAN BUMBAK ET AL: "Best practices in heterotrophic high-cell-density microalgal processes: achievements, potential and possible limitations", APPLIED MICROBIOLOGY AND BIOTECHNOLOGY, SPRINGER, BERLIN, DE, vol. 91, no. 1, 13 mai 2011 (2011-05-13), pages 31-46, XP019915707, ISSN: 1432-0614, DOI: 10.1007/S00253-011-3311-6
- YAGUCHI T ET AL: "PRODUCTION OF HIGH YIELDS OF DOCOSAHEXAENOIC ACID BY SCHIZOCHYTRIUMSP. STRAIN SR21", JOURNAL OF THE AMERICAN OIL CHEMISTS' SOCIETY. (JAOCS), SPRINGER, DE, vol. 74, no. 11, novembre 1997 (1997-11), pages 1431-1434, XP000891774, ISSN: 0003-021X, DOI: 10.1007/S11746-997-0249-Z
- DULCE MARTINS ET AL: "Alternative Sources of n-3 Long-Chain Polyunsaturated Fatty Acids in Marine Microalgae", MARINE DRUGS, vol. 11, no. 7, 27 juin 2013 (2013-06-27), pages 2259-2281, XP055185025, DOI: 10.3390/md11072259
- KAI-CHUANG CHAUNG ET AL: "Effect of culture conditions on growth, lipid content, and fatty acid composition of Aurantiochytrium mangrovei strain BL10", AMB EXPRESS, vol. 2, no. 1, 2012, page 42, XP055078009, ISSN: 2191-0855, DOI: 10.1161/01.ATV.14.4.567
- RENATO S COELHO ET AL: "High Cell Density Cultures of Microalgae under Fed-batch and Continuous Growth", CHEMICAL ENGINEERINGTRANSACTIONS, vol. 38, juin 2014 (2014-06), pages 313-318, XP055223893, ISSN: 2283-9216, DOI: 10.3303/CET1438053

## Description

L'invention se rapporte à un procédé de culture de microalgues du genre *Crypthecodinium* présentant un gène codant la petite sous-unité de l'ARN ribosomique 18s présentant une identité génétique d'au moins 96% avec le même gène de la souche de l'espèce *Crypthecodinium cohnii,* déposée à la CCAP (Culture Collection of Algae and Protozoa), sous le numéro CCAP 1104/3, par ailleurs dénommée dans le présent texte "souche CCAP 1104/3".

Dans le présent texte l'expression "microalgues visées par l'invention" doit s'entendre comme désignant les microalgues présentant un gène codant la petite sous-unité de l'ARN ribosomique 18s présentant une identité génétique d'au moins 96% avec le même gène de la souche de l'espèce *Crypthecodinium cohnii,* déposée à la CCAP (Culture Collection of Algae and Protozoa) sous le numéro CCAP 1104/3 que l'on identifie comme microalgues du genre *Crypthecodinium.*

De même par "souche ", on entend non seulement les souches naturelles de microalgues visées par l'invention, mais également les mutants desdites souches naturelles. Par mutants, on entend un organisme dérivé de la souche originelle dont le patrimoine génétique a été modifié soit par un processus naturel, soit par des méthodes physico-chimiques connues par l'homme de l'art pouvant générer des mutations aléatoires (UV etc...), soit par des méthodes de génie génétique.

Par "milieu de culture chimiquement défini ", on entend un milieu de culture dans lequel la teneur de chaque élément est connue. Précisément, l'invention vise un milieu ne comportant pas de matières organiques riches ou complexes comme par exemple des extraits de levures ou d'autres sources complexes de protéines ou autres matières organiques, telles que de la peptone ou autres matières minérales riches comme par exemple les sels minéraux marins ou autres agents de croissance complexes, n'ayant pas de concentration fixe de chacun de leurs composants.

Par "haute densité cellulaire ", on entend une concentration en biomasse dans le milieu de culture au moins égale à 50 g/L, voire supérieure.

Plus précisément l'invention concerne un procédé de culture desdites microalgues visées par l'invention dans un milieu chimiquement défini pouvant permettre la production à haute densité desdites souches de microalgues visées par l'invention

Le procédé selon l'invention peut en outre permettre d'obtenir une haute densité en biomasse et un enrichissement du contenu desdites microalgues visées par l'invention ainsi cultivées en lipides et plus particulièrement en acide docosahexaénoïque (DHA).

Parmi les acides gras polyinsaturés, certains hautement insaturés (PUFA, PolyUnsatured Fatty Acid) de la série des oméga-3 (PUFA-ω3), en particulier l'acide éicosapentaénoïque (EicosaPentanoïc Acid, EPA ou C20:5 ω3) et l'acide docosahexaénoïque (DocosaHexaénoïc Acid, DHA ou C22:6 ω3), et de la série des oméga-6 (PUFA-ω6), en particulier, l'acide arachidonique (ARachidonic Acid, ARA ou AA ou encore acide eicosatétraénoïque C20:4 ω6), ont une importance nutritionnelle reconnue et présentent de fortes potentialités en terme d'applications thérapeutiques.

Considéré comme nutriment essentiel, le DHA est nécessaire au développement normal et fonctionnel des cellules, et joue un rôle crucial dans divers processus et fonctions biochimiques. Il est indispensable au développement du système nerveux central et à la fonction rétinienne, par incorporation dans les membranes cellulaires, et joue un rôle capital dans l'acquisition et le maintien satisfaisant des mécanismes de la vision et de la mémoire. Le DHA est aussi utilisé dans la composition de produits pharmaceutiques, soit comme principe actif, soit comme vecteur de principes actifs ou encore comme précurseur. On entend par précurseur, une molécule à partir de laquelle il est possible de synthétiser d'autres molécules plus ou moins complexes que ledit précurseur.

Dans le cas d'une utilisation pharmaceutique du DHA, il est nécessaire de fournir une molécule dans son état pur. Il est par exemple nécessaire de réduire au minimum la contamination par de l'acide docosapentaénoique (DPA : DocosaPentaenoic Acid) ou de l'acide éicosapentaénoïque (EPA : EicosaPentaenoic Acid). Ces deux dernières molécules sont souvent produites avec le DHA chez les microalgues. Afin de faciliter cette purification, pour obtenir, si possible facilement, un produit d'une pureté conforme aux exigences réglementaires applicables aux produits alimentaires ou aux produits pharmaceutiques, et cela à moindre coût, il est souhaitable de partir d'une huile dans laquelle le DHA est majoritaire parmi les PUFAs et dans laquelle les teneurs en molécules telles que l'EPA ou encore le DPA sont très faibles.

Le DHA utilisé actuellement dans l'industrie pharmaceutique et dans les produits alimentaires est principalement obtenu à partir de l'huile de poisson.

L'utilisation d'un DHA obtenu à partir d'huile de microalgues, par exemple issues de cultures en bioréacteur, pourrait permettre de s'affranchir des problèmes de contamination des huiles généralement utilisées, par exemple les huiles de poisson, éventuellement contaminées par des polluants de type métaux lourds, pesticides, polychlorobiphényles (PCB), ainsi que des problèmes d'odeur et du manque de stabilité oxydative que peuvent présenter ces huiles, etc...

Les microalgues font l'objet actuellement de nombreux projets industriels car certaines espèces sont capables d'accumuler ou de secréter des quantités importantes de lipides, notamment d'acides gras polyinsaturés.

Les microalgues du genre *Crypthecodinium* sont connues pour leur capacité à produire une huile riche en DHA et contenant de faibles teneurs en DPA, EPA et autres PUFAs [Mendes A, et al. (2008) Crypthecodinium cohnii with emphasis on DHA production: a review. J. Appl. Phycol., 21:199-214]. Le DHA contenu dans cette huile est donc plus facile à purifier comparé, par exemple, au DHA contenu dans une huile de Thraustochytrides de type *Aurantiochytrium* ou *Schyzochytrium* ou encore au DHA contenu dans une huile de poisson.

L'huile produite à partir des microalgues visées par l'invention, est donc particulièrement appropriée pour la production de DHA à destination d'applications pharmaceutiques et nutraceutiques, et pour son utilisation sous forme d'esters de DHA par exemple des esters éthyliques.

Pour mettre en oeuvre la production des acides gras par des microalgues à l'échelle industrielle, plusieurs facteurs doivent être pris en compte pour rendre la production rentable. Parmi ces facteurs, on peut citer :
- les coûts des matières premières et des équipements (leur achat ou location et leur maintenance), ainsi que de la main d'oeuvre ;
- les exigences techniques de la production : par exemple, le nombre et la difficulté technique des étapes de pré-culture et de culture, le suivi en ligne des cultures, et les étapes de traitement de la biomasse issue de la culture pour valoriser le produit ;
- la qualité, la reproductibilité et le rendement des produits issus de la culture ;
- le traitement des effluents issus de la culture.

Les milieux de culture généralement utilisés actuellement dans l'art antérieur pour cultiver les microalgues visées par l'invention, en hétérotrophie ou en mixotrophie, contiennent des quantités significatives de matières organiques riches aussi appelées matières organiques complexes. Par matières organiques riches ou complexes on entend des matières organiques non purifiées, se présentant sous la forme de mélanges pour lesquels la composition exacte et les concentrations des divers composants du mélange ne sont pas connues avec exactitude, pas maitrisées, et peuvent présenter une variabilité significative d'un lot à un autre. Comme exemple de matière organique riche ou complexe, on peut citer les extraits de levure ou les peptones qui sont des produits d'une réaction d'hydrolyse de protéines.

Ces milieux peuvent aussi contenir des matières minérales riches (aussi appelées sels complexes), c'est à dire des matières minérales non purifiées se présentant sous la forme de mélanges dont les concentrations et la composition exacte peuvent présenter une variabilité significative d'un lot à un autre, et ne sont pas maitrisées. A titre d'exemple on peut citer les sels marins.

Par simplification, on utilisera dans le présent texte l'expression "milieu(x) complexe(s)" pour désigner un milieu de culture comprenant des matières organiques riches ou complexes ou des matières minérales riches ou complexes ou les deux.

Plusieurs publications décrivent la production de DHA à haute densité cellulaire dans un milieu complexe avec des souches de microalgues visées par l'invention.

De Swaaf ME, et col [(2003) High-cell-density fed-batch cultivation of the docosahexaenoic acid producing marine alga Crypthecodinium cohnii. Biotechnol Bioeng. 81:666-672 doi:10.1002/bit.10513] décrivent par exemple le milieu complexe suivant : 10 g/L d'extrait de levure ; 25 g/L de sels de mer ; 8 g/L de source carbonée, ladite source carbonée étant de l'acétate de sodium. Les auteurs décrivent une production de 109 g/L de biomasse (Matière sèche) en mode fermentation discontinue alimentée (fed batch) riche en lipides (61%), eux-mêmes constitués de 31% de DHA. La productivité maximale obtenue dans ces conditions est de 38 mg de DHA par litre de milieu et par heure. Par contre, les auteurs décrivent que le problème de viscosité de la culture liée à la sécrétion d'un polysaccharide par la microalgue empêcherait de réaliser la culture à l'échelle industrielle.

WO 2001/004338 décrit un procédé de culture de *Crypthecodinium cohnii* utilisant l'acide acétique comme source principale de carbone dans un milieu de culture complexe comprenant des extraits de levure et des sels marins. Ce procédé permet d'atteindre 45g/L de biomasse, dont 12,3 g/L de lipides en 140h, avec une teneur en DHA dans les lipides de 31%.

EP 0622463 décrit un procédé de culture continue de *Crypthecodinium cohnii* dans un milieu complexe comprenant du glucose et de l'extrait de levure, respectivement comme source principale de carbone et comme source azotée. En mode fermentation discontinue alimentée (fed batch) des productivités de biomasse (matière sèche) de 0,21 g/L/h et de DHA de 0,01g/L/h sont décrites.

Cependant, l'utilisation de milieux complexes pour la culture des microalgues visées par l'invention, dans les procédés de production d'huile et/ou autres molécules d'intérêt présente les inconvénients suivants :
- elle implique des surcoûts en termes de procédés pour le traitement de la biomasse pour arriver au produit final (traitement en aval ou "*down stream processing* "ou "DSP "en anglais),
- elle ne permet pas de maîtriser et de contrôler complètement la composition du milieu de culture, et ainsi la reproductibilité du procédé de culture d'un lot de matière première à l'autre,
- elle limite la capacité à caractériser la composition du milieu complexe et ainsi limite la traçabilité du produit,
- elle entraîne un surcoût par rapport à l'utilisation de milieux chimiquement définis. En effet, les milieux, de type extrait de levure, ont un coût supérieur à un mélange de type azote minéral, minéraux, vitamines.

L'utilisation de milieux complexes pour atteindre de hautes densités cellulaires entraine aussi l'accumulation dans le milieu de culture de substances non utilisées par les microalgues, tel que des minéraux, des protéines, des substances inhibitrices à la croissance des microalgues (acides organiques etc.,), etc.... En effet, les milieux complexes ne sont généralement pas consommés dans leur totalité par les microalgues, qui se contentent de prélever les éléments nécessaires à leurs besoins. La fraction non utilisée s'accumule dans le milieu de culture. Cela peut présenter des inconvénients, particulièrement dans le cas de procédés à haute densité cellulaire, dans les étapes de traitement en aval comme, par exemple, les étapes de séchage de la biomasse et dans les étapes de purification comme, par exemple, les étapes d'ultra-filtration.

L'utilisation de milieux complexes dont la composition exacte n'est pas connue présente en outre un inconvénient pour le procédé. En effet, leur contenu en azote organique, minéraux ou vitamines peut avoir une influence sur le métabolisme des microalgues, et donc sur leur croissance et leur production de molécule(s) d'intérêt.

La composition exacte de ces matières premières n'étant pas garantie, elle est susceptible de varier selon le fournisseur, le produit exact de référence et même d'un lot à l'autre. La reproductibilité de la composition du produit final recherché peut alors varier d'un lot à l'autre. Ceci représente ainsi un obstacle pour la standardisation des produits finaux issus de la biomasse obtenue par culture dans un milieu complexe.

Enfin, comme la composition exacte des milieux complexes n'est pas complètement connue, cela réduit les possibilités de traçabilité et de suivi-qualité du produit. Or, la traçabilité et le suivi-qualité du produit sont importants, voire essentiels, en particulier lorsqu'on envisage une application pharmaceutique dudit produit.

De même, de manière générale, les milieux complexes utilisés actuellement dans l'art antérieur pour cultiver les microalgues visées par l'invention, en hétérotrophie ou en mixotrophie, peuvent contenir également des quantités significatives de sel de mer, dont la composition n'est pas standardisée et donc varient selon le fournisseur, le produit référencé, voire même d'un lot à un autre. Cela peut également rendre difficile la reproductibilité du procédé de culture avec des milieux de culture non définis.

L'utilisation massive de chlorure de sodium (NaCl) pour la culture des microalgues dans les procédés de production d'huile et/ou autres molécules d'intérêt entraîne de plus des surcoûts importants en termes d'investissement, de traitement des effluents et limite la valorisation des coproduits. En effet, les ions chlorure peuvent provoquer une dégradation des dispositifs de culture comme par exemple l'acier inoxydable utilisé dans lesdits dispositifs de culture. Ceci a pour conséquence soit l'utilisation d'alliages particuliers plus résistants aux ions chlorure, mais plus coûteux, soit une dégradation précoce de l'outil de production. Dans les deux cas, les coûts d'investissement sont substantiellement accrus.

Par ailleurs, l'utilisation massive de chlorure de sodium (ou d'autres sels de sodium, notamment de sulfate de sodium et de carbonate de sodium) entraîne des surcoûts importants en terme de traitement des effluents notamment dans la désalinisation de l'eau.

Enfin, la présence d'excès de sels de sodium dans les coproduits de type tourteaux constitués par la biomasse restante après extraction de l'huile, peut rendre difficile leur valorisation, notamment pour la nutrition animale.

Le remplacement de ces sels par un mélange de minéraux connus, élaboré à partir de minéraux purifiés, permet une meilleure maîtrise du procédé et une meilleure traçabilité des matières premières.

Ce remplacement des sels, particulièrement du sel de mer, par un mélange connu peut permettre également de réduire substantiellement la quantité de NaCl présente dans le milieu pour l'ajuster éventuellement aux stricts besoins de la souche et ainsi réduire les problèmes liés à la présence du chlorure de sodium (NaCl) dans le milieu.

La formulation de milieux chimiquement définis pour la culture de souches de microalgues visées par l'invention, a été décrite à plusieurs reprises dans la littérature. En 1957, Provasoli et Gold ont publié le premier milieu dit "synthétique" pour une souche de *Crypthecodinium cohnii* [Provasoli, L., J. J. A. McLaughlin and M. R. Droop: The development of artificial media for marine algae. Arch. Mikrobiol. 25, 392-428 (1957)].

Depuis cette date, d'autres groupes ont encore publié sur le sujet. En 1993, Nerad rapporte la création du milieu A₂E₆, notamment utilisé aujourd'hui pour la conservation de souches en banque (ATCC 460) [Nerad TA (1993) American type culture collection, catalogue of protists, 18th edn. Rockville, USA ATCC Médium: 460 A2E6 Médium], et dont la recette est disponible sur le site internet de l'ATCC.

Plus récemment, US 2008/0032352 A1 décrit un procédé de culture hétérotrophique avec un milieu chimiquement défini visant notamment à réduire la concentration en ions chlorure.

Toutefois, dans l'art antérieur qui décrit la croissance de souches de microalgues visées par l'invention, dans un milieu synthétique, les auteurs ne parviennent pas à atteindre de hautes densités cellulaires. Les concentrations en biomasse obtenues sont inférieures à 50 g de matière sèche par litre de milieu.

Ainsi, ces milieux décrits dans l'art antérieur semblent plus convenir au maintien de la souche qu'à une production à fortes concentrations en biomasse (supérieure à 50 g/L), et ne semblent donc pas adaptés à une industrialisation du procédé.

Il existe donc toujours un besoin de fournir des milieux de culture synthétiques dont la composition est bien définie et bien maîtrisée, qui sont reproductibles et qui permettent d'atteindre de hautes densités cellulaires.

Il est souhaitable de pouvoir cultiver les souches de microalgues visées par l'invention, sans utiliser de milieux complexes, dans des conditions optimales pour maximiser le rendement de l'acide gras à produire, tout en réduisant les coûts de la production pour la fermentation, en facilitant le traitement en aval, en améliorant la maîtrise du procédé et sa reproductibilité et en améliorant la traçabilité et le suivi de la qualité tout au long de la chaine de valeur.

Ceci est notamment important dans le cadre d'une application pharmaceutique, la maîtrise de la qualité des matières premières et de la reproductibilité du procédé sont des paramètres critiques, tandis que la maîtrise du milieu et du procédé permettent d'obtenir une huile très concentrée en DHA ce qui facilite les étapes de purification.

En particulier, il est souhaitable de fournir des méthodes de culture des souches de microalgues visées par l'invention, qui permettent de substantiellement réduire, voire éliminer, l'utilisation de milieux complexes et en particulier des sels marins.

Dans le contexte de la présente invention, il est souhaitable d'obtenir des densités en biomasse et en lipides suffisantes pour une production industrielle du DHA. Il pourrait donc être souhaitable d'obtenir des concentrations, par exemple, supérieures à 50 g/L de matière sèche, de préférence supérieures à 150 g/L et plus préférentiellement encore supérieures à 200 g/L de matière sèche.

Il pourrait être souhaitable non seulement de pouvoir produire une haute densité cellulaire, mais aussi des quantités de DHA suffisantes pour une production de DHA à l'échelle industrielle. Il peut être aussi souhaitable d'obtenir des proportions en autres PUFAs, tels que le DPA, au moins inférieures à 5%, de préférence au moins inférieures à 2%, de préférence encore au moins inférieures à 1%, encore plus préférentiellement inférieure à 0,8%, avantageusement au moins inférieures à 0,5%, ou encore au moins inférieures à 0,1%, très avantageusement de préférence au moins inférieures à 0,01%.

Ce sont quelques-uns des objectifs que la Demanderesse s'est fixés et que la présente invention vise à atteindre.

Ainsi, la Demanderesse a mis au point un milieu chimiquement défini, adapté à la culture à haute densité cellulaire de souches de microalgues visées par l'invention. Le procédé mis au point peut permettre notamment d'atteindre une concentration en biomasse équivalente ou supérieure à celles décrites dans l'art antérieur dans un milieu complexe, tout en conservant une productivité compétitive par rapport à ces dernières cultures.

L'utilisation d'un milieu chimiquement défini selon l'invention peut permettre notamment de contrôler la quantité de chaque élément présent dans le milieu.

Ainsi, en utilisant ce milieu chimiquement défini, les inventeurs ont mis au point un nouveau procédé de culture à haute densité cellulaire de microalgues visées par l'invention.

De plus, selon un mode de réalisation particulier de l'invention, le procédé de culture peut permettre d'orienter le métabolisme des souches vers une production de DHA améliorée. Cela peut être accompli en contrôlant les apports en nutriments, et notamment les apports en source carbonée, azotée et phosphatée. Par ce procédé, les inventeurs ont réussi à augmenter d'au moins 30%, voire jusqu'à plus de 50%, la teneur en DHA dans les lipides produits par les souches de microalgues visées par l'invention, en comparaison avec la culture en milieu complexe, tout en maintenant la concentration des autres PUFAs à une valeur faible, avantageusement au moins inférieure à 5%, très avantageusement inférieure à 1 %, préférentiellement au moins inférieure à 0,8%.

L'huile ainsi produite par le procédé de l'invention peut être fortement enrichie en DHA, ce qui constitue une amélioration significative en comparaison avec les procédés de l'art antérieur.

Par ailleurs, les coûts liés au traitement en aval sont réduits par rapport aux procédés de l'art antérieur.

Ainsi, la Demanderesse est parvenue à mettre au point un milieu de culture chimiquement défini, c'est-à-dire de manière générale, sans milieux riches ou complexes et sans matières minérales riches ou complexes, particulièrement sans sels marins.

Le milieu selon l'invention peut permettre de cultiver les souches de microalgues visées par l'invention, et peut permettre d'obtenir une production à haute densité en biomasse desdites microalgues qui peut par exemple être au moins supérieure à 50 g/L, de préférence à 100 g/L, 150 g/L, 200 g/L, ou 250 g/L, plus préférentiellement 270 g/L en matière sèche.

Les taux de DHA dans la culture réalisée selon le procédé selon l'invention peuvent être par exemple au moins supérieurs à 5 g/L ou 10 g/L, de préférence supérieurs à 20 g/L, et plus préférentiellement encore supérieurs à 30 g/L, tout en maintenant la production des autres PUFAs à par exemple moins de 1 % de la teneur totale en acide gras, préférentiellement moins de 0,8% de la teneur totale en acide gras.

L'invention concerne donc la culture de souches de microalgues du genre *Crypthecodinium* présentant un gène codant la petite sous-unité de l'ARN ribosomique 18s présentant une identité génétique d'au moins 96% avec le même gène de la souche de l'espèce *Crypthecodinium cohnii,* déposée à la CCAP (Culture Collection of Algae and Protozoa), sous le numéro CCAP 1104/3.

La souche de référence, *Crypthecodinium cohnii,* déposée à la CCAP (Culture Collection of Algae and Protozoa), sous le numéro CCAP 1104/3 appartient au Clade 2 comme décrit dans l'article : [Prabowo DA, Hiraishi O, and Suda S. DIVERSITY OF Crypthecodinium spp. (DINOPHYCEAE) FROM OKINAWA PREFECTURE, JAPAN. Journal of Marine Science and Technology, Vol. 21, Suppl., pp. 181-191 (2013)].

A titre d'exemple non limitatif il est possible de citer comme microalgues visées afin de mettre en pratique l'invention les souches des espèces *Crypthecodinium sp* D31, *Crypthecodinium sp* SS2-2, *Crypthecodinium sp* SZ13-3, *Crypthecodinium sp* SZ13-2, *Crypthecodinium sp* SZ13-1, *Crypthecodinium cohnii* (Numéro d'accession dans Gene Bank : M64245), *Crypthecodinium cohnii* (Numéro d'accession dans Gene Bank : FJ821501), *Crypthecodinium sp* OKI5-1, *Crypthecodinium sp* SZ7-1, *Crypthecodinium sp* ISG40-1, *Crypthecodinium sp* ISK1-2, *Crypthecodinium sp* ISK1-1, *Crypthecodinium cohnii* (Numéro d'accession dans Gene Bank : DQ241737) ou encore les souches déposées par la Demanderesse auprès de la CCAP *Crypthecodinium cohnii* déposée sous le numéro CCAP 1104/3, *Crypthecodinium cohnii* déposée sous le numéro CCAP 1104/5, ou encore *Crypthecodinium cohnii* déposée sous le numéro CCAP 1104/4.

Le tableau 1 ci-dessous présente le pourcentage d'identité des gènes codant la petite sous-unité de l'ARN ribosomique 18s de différentes souches présentant une identité génétique d'au moins 96% avec le même gène de la souche l'espèce *Crypthecodinium cohnii,* déposée à la CCAP (Culture Collection of Algae and Protozoa), sous le numéro CCAP 1104/3, des souches évoquées ci-dessus.

**Tableau 1**

| Souches | Numéro d'accès de la séquence comparée dans GENBANK | Longueur des séquences comparées (%) | % d'identité avec la référence |
|---|---|---|---|
| *Crypthecodinium sp* D31 | AB811790 | 100% | 99% |
| *Crypthecodinium sp* SS2-2 | AB871552 | 98% | 99% |
| *Crypthecodinium sp* SZ13-3 | AB871549 | 98% | 99% |
| *Crypthecodinium sp* SZ13-2 | AB871548 | 98% | 99% |
| *Crypthecodinium sp* SZ13-1 | AB871547 | 98% | 99% |
| *Crypthecodinium cohnii* | M64245 | 100% | 99% |
| *Crypthecodinium cohnii* | FJ821501 | 100% | 99% |
| *Crypthecodinium sp* OKI5-1 | AB871551 | 98% | 99% |
| *Crypthecodinium sp* SZ7-1 | AB871550 | 98% | 99% |
| *Crypthecodinium sp* ISG40-1 | AB871546 | 98% | 99% |
| *Crypthecodinium sp* ISK1-2 | AB871545 | 98% | 99% |
| *Crypthecodinium sp* ISK1-1 | AB871544 | 98% | 99% |
| *Crypthecodinium cohnii* | DQ241737 | 91% | 96% |

La présente invention a donc pour objet un procédé de culture de souches de microalgues visées par l'invention, en hétérotrophie ou en mixotrophie, dans un milieu chimiquement défini. Ce procédé de culture permet d'obtenir de hautes densités en biomasse, en lipides, et spécifiquement en DHA, tout en maintenant une faible teneur en DPA.

Les souches de microalgues pour la mise en pratique de l'invention sont capables de croître dans un milieu de culture chimiquement défini mis au point par la Demanderesse.

L'invention a donc pour objet un procédé de culture de microalgues, dans un milieu chimiquement défini, pouvant comprendre au moins des macroéléments, des microéléments et des vitamines auxquels est ajoutée une source de carbone.

Plus précisément le milieu de culture selon l'invention peut comprendre, outre la source de carbone, à titre de macroéléments du chlorure de sodium (NaCl), du chlorure de magnésium, avantageusement sous forme d'hexahydrate (MgCl₂ 6H₂O), du bicarbonate de sodium (NₐHCO₃), de l'hydrogénophosphate de potassium (K₂HPO₄), du chlorure de calcium, avantageusement sous forme dihydrate (CaCl₂ 2H₂O), du chlorure de fer (III) (FeCl₃ 6H₂O), de l'acide borique (H₃BO₃) et une source d'azote qui peut être un sel de nitrate comme par exemple le nitrate de potassium (KNO₃) le nitrate de sodium (NaNO₃), le nitrate de calcium (Ca(NO₃)₂) ou un sel de glutamate ou un sel d'ammonium ou de l'urée.

Selon l'invention, le chlorure de sodium peut être à une concentration comprise entre 2 et 8 g/L, préférentiellement entre 4 et 6 g/L.

Selon l'invention, l'hydrogénophosphate de potassium peut être à une concentration comprise entre 0,3 et 0,7 g/L, préférentiellement entre 0,4 et 0,6 g/L.

Selon l'invention, le chlorure de magnésium peut être à une concentration comprise entre 0,6 et 1,4 g/L, avantageusement entre 0,8 et 1,2 g/L.

Selon l'invention, le bicarbonate de sodium peut être à une concentration comprise entre 0,14 et 0,26 g/L, avantageusement entre 0,16 et 0,24 g/L.

Selon l'invention, la source d'azote peut être à une concentration comprise entre 1,7 et 3,1 g/L, avantageusement entre 1,9 et 2,9 g/L.

Selon l'invention, le chlorure de calcium peut être à une concentration comprise entre 1,4 et 2,6 g/L, avantageusement entre 1,6 et 2,4 g/L.

Selon l'invention, le chlorure de fer (III) peut être à une concentration comprise entre 0,016 et 0,024 g/L, avantageusement entre 0,018 et 0,022 g/L.

Selon l'invention, l'acide borique peut être à une concentration comprise entre 0,046 et 0,74 g/L, avantageusement entre 0,048 et 0,72g/L.

De même le milieu de culture dans le procédé selon l'invention peut comprendre à titre de microéléments de l'acide éthylène diamine tétraacétique sous forme disodique (Na₂EDTA), du chlorure de manganèse (II) tetrahydrate (MnCl₂ 4H₂O), du chlorure de zinc (ZnCl₂), du chlorure de cobalt hexahydrate (CoCl₂ 6H₂O), du sulfate de cuivre pentahydrate (CuSO₄ 5H₂O), et du sulfate de nickel hexahydrate (NiSO₄ 6H₂O).

Selon l'invention, l'acide éthylène diamine tétraacétique sous forme disodique peut être à une concentration comprise entre 0,01 et 0,08 g/L, avantageusement entre 0,015 et 0,06 g/L.

Selon l'invention, le chlorure de manganèse (II) tetrahydrate peut être à une concentration comprise entre 0,001 et 0,02 g/L, avantageusement entre 0,002 et 0,01 g/L.

Selon l'invention, le chlorure de zinc peut être à une concentration comprise entre 0,0001 et 0,0008 g/L, avantageusement entre 0,00015 et 0,0006 g/L.

Selon l'invention, le chlorure de cobalt hexahydrate peut être à une concentration comprise entre 0,00005 et 0,0005 g/L, avantageusement entre 0,00007 et 0,0003 g/L.

Selon l'invention, le sulfate de cuivre pentahydrate peut être à une concentration comprise entre 0,00007 et 0,004 g/L, avantageusement entre 0,0009 et 0,0036 g/L.

Selon l'invention, le sulfate de nickel hexahydrate peut être à une concentration comprise entre 0,0005 et 0,003 g/L avantageusement entre 0,0007 et 0,0028 g/L.

De même le milieu de culture dans le procédé selon l'invention peut comprendre à titre de vitamine de la biotine, de la thiamine, de la cobalamine ou vitamine B12 et du panthoténate ou vitamine B5.

Selon l'invention, la biotine peut être à une concentration comprise entre 0,5.16⁻⁶ et 1,5.10⁻⁴ g/L, avantageusement entre 1x10⁻⁶ et 1x10⁻⁴ g/L.

Selon l'invention, la thiamine peut être à une concentration comprise entre 0,5.16⁻⁶ et 1,5.10⁻⁴ g/L, avantageusement entre 1x10⁻⁶ et 1x10⁻⁴ g/L.

Selon l'invention, la cobalamine peut être à une concentration comprise entre 0,5.16⁻⁶ et 1,5.10⁻⁴ g/L, avantageusement entre 1x10⁻⁶ et 1x10⁻⁴ g/L.

Selon l'invention, le panthoténate peut être à une concentration comprise entre 0,5.16⁻⁶ et 1,5.10⁻⁴ g/L, avantageusement entre 1x10⁻⁶ et 1x10⁻⁴ g/L.

Selon l'invention, le milieu de culture défini peut comprendre une source carbonée. A titre d'exemple de source carbonée utilisable dans le milieu défini selon l'invention peut être un (ou des) glucide(s), un (ou des) acétate(s), un ou des alcools ou une ou des molécules complexes. Avantageusement la source carbonée peut être un mélange de sources carbonées. Par exemple les produits issus de la biotransformation de l'amidon, par exemple à partir de maïs, de blé ou de pomme de terre, notamment les hydrolysats de l'amidon, qui sont constitués de molécules de petite taille, constituent des substrats carbonés utilisables selon l'invention.

A titre d'exemple de glucides utilisables dans le milieu défini selon l'invention on peut citer le saccharose (ou sucrose), le lactose, l'amidon, l'amylose, l'amylopectine, la cellulose ou ses dérivés, le glycogène, la dihydroxyacétone, le glycéraldéhyde, l'érythrose, le thréose, l'érythrulose, le ribose, le ribofuranose , l'arabinose, le xylose, le lyxose, le ribulose, la xylulose, l'allose, l'altrose, le glucose, le glucopyranose, le mannose, le gulose, l'idose, le galactose, le galactopyranose, le talose, le psicose, le fructose, le fructofuranose, le sorbose, le tagatose, le sédoheptulose, le 2-désoxyribofuranose, le tréhalose, le maltose, le cellobiose, l'isomaltose, le raffinose, le gentianose, l'agar-agar, l'acide hyaluronique, l'orthonitrophényl-β-D-galactopyranoside (ONPG).

A titre d'exemple d'acétate on peut citer l'acétate de sodium ou l'acide acétique.

A titre d'exemple d'alcool on peut citer l'éthanol ou le glycérol.

Préférentiellement selon l'invention la source carbonée pourra être du glycérol.

Selon l'invention, la concentration en source carbonée peut être comprise entre 10 mM et 500 mM. Cependant l'homme du métier saura adapter la concentration en source carbonée selon sa nature et selon les besoin de la souche cultivée. Par exemple le glucose, le glycérol et l'éthanol pourront avoir une concentration comprise entre 200 et 500 mM. Si la source de carbone est de l'acétate, par exemple de l'acétate de sodium ou de l'acide acétique, leurs concentrations dans le milieu de culture selon l'invention pourront être comprises entre 10 et 100 mM.

Selon une variante de l'invention, le milieu défini chimiquement peut n'être constitué que des éléments décrits ci-dessus auxquels une source de carbone telle que décrite précédemment est ajoutée.

L'invention concerne un procédé de culture de souches de microalgues, avantageusement les microalgues du genre *Crypthecodinium,* présentant un gène codant la petite sous-unité de l'ARN ribosomique 18s présentant une identité génétique d'au moins 96% avec le même gène de la souche de l'espèce *Crypthecodinium cohnii,* déposée à la CCAP (Culture Collection of Algae and Protozoa), sous le numéro CCAP 1104/3, caractérisé en ce que qu'il peut comprendre au moins une première étape dans laquelle on cultive lesdites souches dans un milieu défini tel que décrit précédemment pendant un temps suffisant pour atteindre une concentration en souches comprise entre 5.10⁶ et 5.10⁸ cellules/mL, avantageusement entre 10⁷ et 10⁸ cellules/mL, et au moins une deuxième étape dans laquelle on ajoute au milieu de culture, simultanément ou successivement, une ou plusieurs solution(s) d'enrichissement en source carbonée comprenant à la fois la source de carbone, la source d'azote et la source de phosphore (solution d'enrichissement C/N/P). Avantageusement selon l'invention la solution d'enrichissement en source carbonée peut être constituée d'une solution comprenant une source carbonée identique ou différente à la source carbonée utilisée à la première étape.

Selon l'invention le temps suffisant pour atteindre une concentration en souches comprise entre 5.10⁶ et 5.10⁸ cellules/mL, avantageusement entre 10⁷ et 10⁸ cellules/mL peut être compris entre 24h et 168h avantageusement entre 24h et 96h. Toutefois l'homme du métier saura aisément déterminer la durée du temps suffisant pour atteindre une concentration en souches comprise entre 5.10⁶ et 5.10⁸ cellules/mL, en analysant régulièrement la concentration en cellules dans le milieu de culture. Toute méthode de mesure de la concentration en cellule dans le milieu de culture peut être utilisée par l'homme du métier pour surveiller l'état d'avancement de la culture comme par exemple le dénombrement cellulaire sur cellule de Mallassez, ou encore avec un compteur à particules comme un "Z Coulter Counter ®" de la marque Beckman ®.

La deuxième étape du procédé selon l'invention a pour but de compenser la consommation en source carbonée, en sources d'azote, et source de phosphore par les cellules en culture au cours de la première étape.

Selon une variante de l'invention, le procédé pourra comprendre une troisième étape dans laquelle on ajoute au milieu de culture, simultanément ou successivement, une ou plusieurs solution(s) d'enrichissement en source de microéléments, et/ou en source de vitamines, avantageusement en source d'azote et/ ou en source de phosphore.

Selon cette variante de l'invention, cette troisième étape a pour but de compenser la consommation en sources de macroéléments, de microéléments et/ou de vitamines par les cellules en culture au cours des premières étapes.

Les inventeurs ont pu montrer que l'ajout à la deuxième étape d'une solution comprenant à la fois la source de carbone, la source d'azote et la source de phosphore mais dont les rapports de concentrations molaires en atomes de carbone/azote/phosphore sont parfaitement définis permet d'orienter le métabolisme des souches en culture préférentiellement vers la formation de DHA. Les inventeurs ont montré, après avoir effectué de nombreuses expériences et essais de culture dans des conditions différentes que cela peut être possible en créant une limitation définie en azote et phosphore.

La production de DHA par les cellules peut augmenter si le substrat additionnel est ajouté sous forme de solution nutritive avec un ratio molaire en atomes de carbone, d'azote et de phosphore défini dans lesquels le phosphore est limité par rapport à l'azote et au carbone et dans lesquels l'azote est limité par rapport au carbone.

Ainsi selon l'invention, la solution d'enrichissement de type C/N/P pourra présenter à quantité de carbone égale jusqu'à deux fois plus ou deux fois moins d'azote, et/ou jusqu'à deux fois plus ou deux fois moins de phosphore par rapport au ratio 891 :26 :1.

Ainsi, la solution d'enrichissement de type C/N/P peut être utilisée aux ratios molaires suivants : 891 :52 :1, 891 :26 :1 et 891 :13 :1, 445 :26 :1, 445 :13 :1 et 445 :6,5 :1, 1782 :104 :1, 1782 :52 :1, et 1782 :26 : 1.

Préférentiellement la solution d'enrichissement de type C/N/P pourra présenter les ratios molaires suivants : 891 :26 :1.

Bien évidemment le procédé selon l'invention peut comprendre une ou plusieurs répétitions des étapes 2 et 3 et ce quel que soit l'ordre dans lequel elles ont été réalisées initialement.

L'homme du métier sait déterminer la quantité et la fréquence des ajouts selon l'état d'avancement de la culture. En début de culture, les éléments présents dans le milieu chimiquement défini, tel que décrit précédemment, permettent de subvenir à l'ensemble des besoins nutritifs des souches en culture. Au stade où la culture a atteint une densité cellulaire comprise entre 5.10⁶ et 5.10⁸, encore plus précisément une densité cellulaire comprise entre 10⁷ et 10⁸ cellules/mL, des substrats carbonés peuvent être ajoutés. De plus, en fonction du mode de réalisation du procédé, des ajouts de solutions nutritives chimiquement définies telles que décrites précédemment, peuvent être réalisés quand la culture atteint une densité cellulaire comprise entre 5.10⁶ et 5.10⁸, encore plus précisément une densité cellulaire comprise entre 10⁷ et 10⁸ cellules/mL. Ces solutions nutritives peuvent notamment constituer des sources d'azote ou de phosphore.

Selon l'invention le pH du milieu de culture pourra être compris entre 3 et 9, avantageusement entre 4 et 8.

De même selon l'invention la température de la culture pourra être comprise entre 15 et 35°C, préférentiellement entre 20 et 30°C.

Selon l'invention, la concentration en oxygène dissous peut être régulée entre 4% et 40% avantageusement entre 5% et 30% au cours de la culture.

Bien entendu, tout autre adjuvant nécessaire à la culture de souches de microalgues, particulièrement celles visées par l'invention, bien connu de l'homme du métier peut être ajouté selon le procédé selon l'invention. Par exemple de l'antimousse peut être ajouté en cours de culture, par exemple 200ppm de Biospumex 153K®, disponible chez Cognis, France.

Les souches visées par l'invention peuvent être cultivées selon les procédés décrits précédemment, puis récupérées pour toute utilisation subséquente envisageable. Préférentiellement, les souches visées par l'invention peuvent être utilisées pour en extraire le contenu lipidique, en particulier le DHA. Les méthodes d'extraction sélective des lipides, dont le DHA, sont connues de l'homme du métier et sont, par exemple, décrites dans l'article de Bligh, E.G. et Dyer, W.J. (1959) [A rapid method of total lipid extraction and purification, Can. J. Biochem. Physiol., 37:911-917].

Le procédé selon l'invention peut permettre d'obtenir une biomasse dont les souches peuvent avoir ainsi une productivité de DHA d'au moins 0,02 g/L/h, de préférence d'au moins 0,03 g/L/h, plus préférentiellement d'au moins 0,05 g/L/h, et plus préférentiellement d'au moins 0,1 g/L/h.

De même le procédé selon l'invention peut permettre d'obtenir une biomasse dont les souches peuvent présenter une teneur en DPA dans les acides gras issus du procédé est inférieure à 3%, de préférence inférieure à 1%, plus préférentiellement inférieure à 0,5%, plus préférentiellement encore inférieure à 0,1%, voire à 0,01%.

L'invention a aussi pour objet un procédé de préparation de DHA, caractérisé en ce que l'on cultive des souches visées par l'invention dans un milieu de culture tel que défini précédemment.

La biomasse obtenue par le procédé selon l'invention peut être soumise à tout autre procédé connu pouvant conduire à la séparation de l'huile contenue dans les souches.
Par exemple après la fermentation la biomasse obtenue peut être concentrée, si nécessaire, à environ 250 g/L. Cette étape a pour but de concentrer la biomasse de souches visées par l'invention afin de diminuer la quantité d'eau à évaporer lors de l'étape suivante. La concentration de la biomasse peut être réalisée par centrifugation. L'intensité de la pesanteur artificielle appliquée peut être supérieure à 4000 g. La biomasse récoltée dans les boues de centrifugation peut être concentrée, à minima, à 250 g/L. Les effluents, constitués majoritairement du milieu de culture, peuvent être éliminés.

L'étape de séchage, qui suit l'étape de concentration, a pour but de préparer les boues de centrifugation à une étape d'extraction mécanique de l'huile. Pour cela, l'étape de séchage peut consister à éliminer l'eau pour obtenir une matière sèche pouvant comprendre un taux d'humidité relative à entre 3 et 5 % (de 95 à 97 % de matière sèche). Cette étape de séchage peut être réalisée par exemple via une technologie de sécheur à cylindre ou par d'autres méthodes connues de l'homme de métier. Les paramètres pour l'étape de séchage pourront être optimisés en fonction de l'humidité relative des boues de fermentation. Par ailleurs, les paramètres de séchage peuvent être également optimisés en corrélant la température des cylindres (contrôle de la pression de la vapeur au-delà de 3 bars) au temps de contact des boues sur les cylindres (vitesse de rotation des cylindres, 2 Hz).

L'huile contenue dans la matière sèche obtenue par l'étape de séchage précédente peut être extraite par toutes méthodes connues de l'homme du métier, par exemple par une technologie classique d'extrusion-pression. Cette extraction mécanique (étape de pressage) peut généralement être réalisée par une presse constituée d'une vis sans fin appliquant une pression sur une cage fermée d'une tuyère. Ce dispositif permet d'obtenir une huile de pression dite "à froid" ou, si elle est réalisée avec un système de collier chauffant "à température contrôlée". Dans tous les cas, l'huile récupérée mécaniquement n'est jamais en contact avec des solvants. Le pressage mécanique de la matière sèche permet d'obtenir une fraction huileuse riche en pigments, et un tourteau constitué entre 3 et 5 % d'huile résiduelle. A ce stade il est possible de délipider entièrement le tourteau, et ainsi récupérer toute l'huile produite au cours de la fermentation. On soumet alors le tourteau à une étape supplémentaire d'extraction par solvant.

L'huile de pression récupérée de l'étape de pressage peut être clarifiée par des méthodes classiques, comme la centrifugation, afin d'éliminer les fines particules solides issues de la pression de la biomasse et en suspension dans l'huile (fines). On obtient alors une huile clarifiée.

Les fines peuvent représenter environ 5 % de l'huile de pression obtenue à partir de matière sèche. L'huile présente dans les fines peut, à l'égal de l'huile résiduelle dans le tourteau, être récupérée par une extraction par solvant.

L'huile clarifiée peut être ensuite transformée, selon des méthodes connues de l'homme du métier, en esters éthyliques d'acides gras par transestérification éthanolique en milieu basique. Ainsi, les acides gras peuvent être séparés du glycérol résidu de la transestérification. Le glycérol peut ensuite être retiré par un lavage à l'eau. L'eau résiduelle présente dans les esters éthyliques peut être de manière générale évaporée par distillation sous vide, par exemple à l'aide d'un Rotavapor®.

Une fois dépourvus d'eau, les esters éthyliques d'acides gras peuvent être séparés selon des méthodes connues de l'homme du métier, par exemple, par distillation moléculaire. Lors de cette étape, trois fractions peuvent, en général, être obtenues:
- une fraction légère, constituée des esters éthyliques d'acides gras à moyennes et longues chaînes (de l'ester éthylique d'acide caprique ou EE-C10:0 à l'ester éthylique de l'acide oléique ou EE-C18:1n9),
- une fraction lourde, constituée des esters éthyliques à très longues chaînes (ester éthylique de DHA ou EE-DHA) ;
- et, selon le mode de culture, une fraction constituée de pigments concentrés (α-Carotène et β-Carotène).

L'EE-DHA peut présenter une pureté supérieure à 98% après l'étape de distillation moléculaire.

L'EE-DHA produit par le procédé de l'invention peut comprendre moins de 1%, voire moins de 0,5% de DPA n6. L'EE-DHA peut donc être directement utilisé comme constituant dans des compositions pharmaceutiques de médicament à visée thérapeutique.

La présente divulgation concerne également une biomasse de microalgues présentant un gène codant la petite sous-unité de l'ARN ribosomique 18s présentant une identité génétique d'au moins 96% avec le même gène de la souche de l'espèce *Crypthecodinium cohnii,* déposée à la CCAP (Culture Collection of Algae and Protozoa), sous le numéro CCAP 1104/3, susceptible d'être obtenue par le procédé selon l'invention, caractérisé en ce qu'elle présente une densité au moins supérieure à 50 g/L, de préférence à 100 g/L, 150 g/L, 200 g/L, ou 250 g/L, plus préférentiellement 270 g/L en matière sèche, ladite biomasse présentant en outre un taux de DHA au moins supérieur à 5 g/L ou 10 g/L, de préférence supérieurs à 20 g/L, et plus préférentiellement encore supérieurs à 30 g/L et éventuellement un taux de PUFAs inférieur à 1 % de la teneur totale en acide gras, préférentiellement inférieur à 0,8 % de la teneur totale en acide gras.

D'autres avantages et particularités de l'invention apparaitront à la lecture des exemples décrits ci-après ainsi que des figures annexées dans lesquelles :

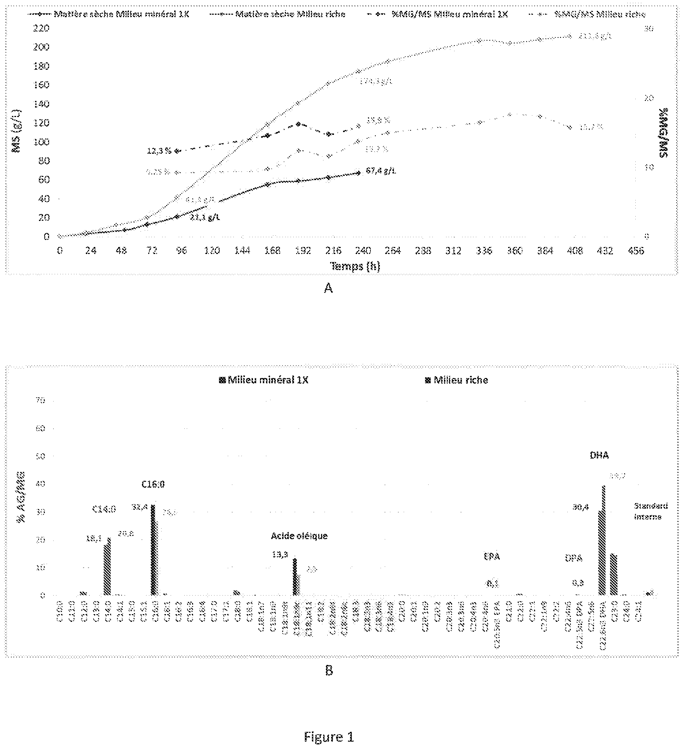

La Figure 1 présente les résultats obtenus au cours de la culture d'une souche de l'espèce *Crypthecodinium cohnii* (souche CCAP 1104/3) selon le procédé de l'invention en milieu chimiquement défini selon l'invention avec ajout d'une solution d'enrichissement en source carbonée, en comparaison d'une culture en milieu riche (Exemple 1). La Figure 1A présente l'évolution de la quantité de matière sèche produite au cours de l'essai. La Figure 1B présente le profil lipidique de l'huile contenue dans la souche CCAP 1104/3 en fin de culture.

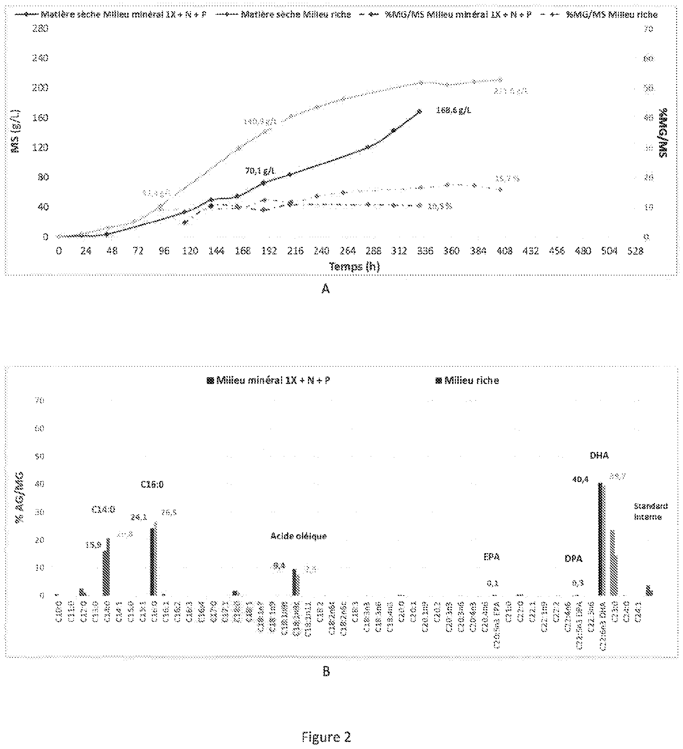

La figure 2 présente les résultats obtenus au cours de la culture d'une souche de l'espèce *Crypthecodinium cohnii* (souche CCAP 1104/3) selon le procédé de l'invention en milieu chimiquement défini selon l'invention avec ajout d'une solution d'enrichissement de source carbonée et d'une solution d'enrichissement de source d'azote et de phosphore en comparaison d'une culture en milieu riche (Exemple 2). La Figure 2A présente l'évolution de la quantité de matière sèche produite au cours de l'essai. La Figure 2B présente le profil lipidique de l'huile contenue dans la souche CCAP 1104/3 en fin de culture.

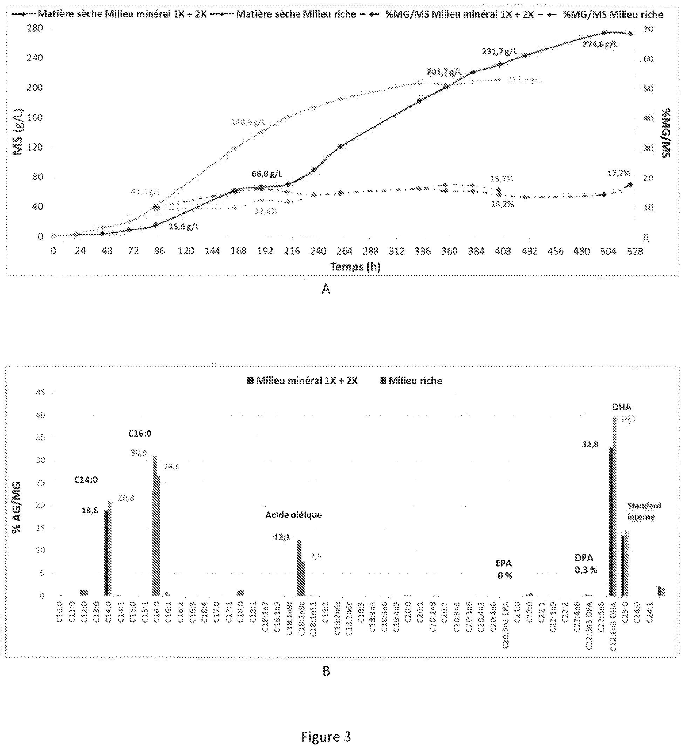

La figure 3 présente les résultats obtenus au cours de la culture d'une souche de l'espèce *Crypthecodinium cohnii* (souche CCAP 1104/3) selon le procédé de l'invention en milieu chimiquement défini selon l'invention avec ajout d'une solution d'enrichissement en source carbonée et d'une solution d'enrichissement en macroéléments, contenant notamment de l'azote et du phosphore, en microéléments et en vitamines, qui permet d'apporter l'équivalent de deux fois la quantité en éléments présente dans le milieu défini utilisé initialement pour un apport total en minéraux et vitamines correspondant à trois fois la quantité initiale ("3X "total) après ajouts, en comparaison d'une culture en milieu riche (Exemple 3). La Figure 3A présente l'évolution de la quantité de matière sèche produite au cours de l'essai. La Figure 3B présente le profil lipidique de l'huile contenue dans la souche CCAP 1104/3 en fin de culture.

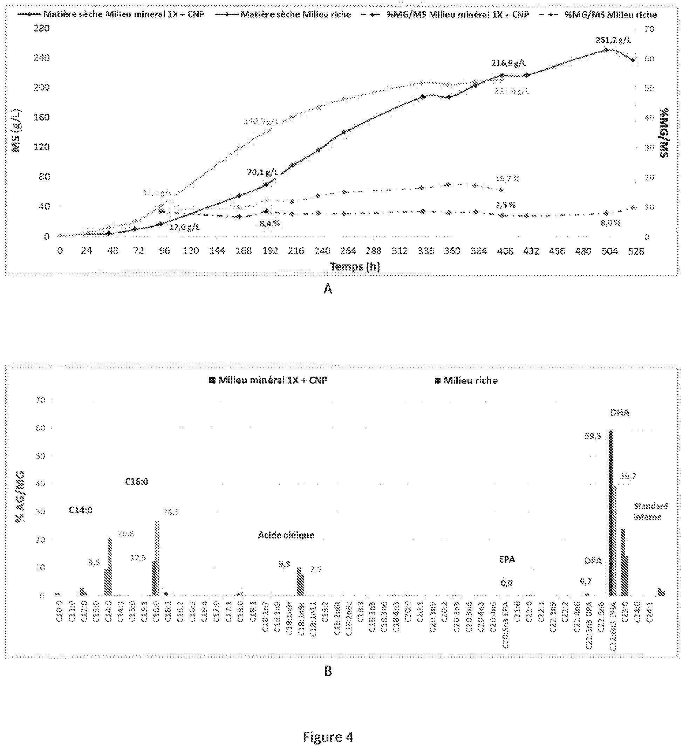

La figure 4 présente les résultats obtenus au cours de la culture d'une souche de l'espèce *Crypthecodinium cohnii* (souche CCAP 1104/3) selon le procédé de l'invention en milieu chimiquement défini selon l'invention avec ajout d'une solution d'enrichissement de type C/N/P, en comparaison d'une culture en milieu riche (Exemple 4). La Figure 4A présente l'évolution de la quantité de matière sèche produite au cours de l'essai. La Figure 4B présente le profil lipidique de l'huile contenue dans la souche CCAP 1104/3 en fin de culture.

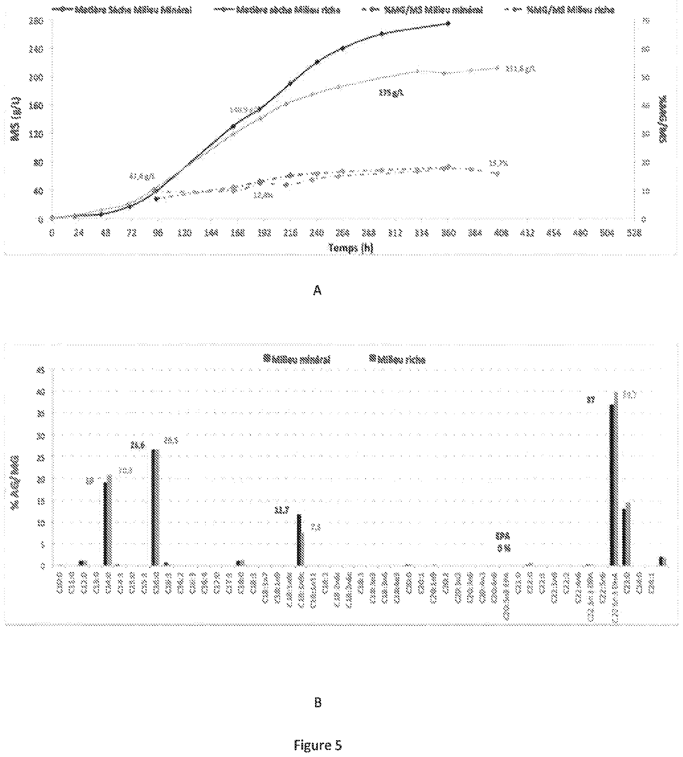

La figure 5 présente les résultats obtenus au cours de la culture d'une souche de l'espèce *Crypthecodinium cohnii* (souche CCAP 1104/3) selon le procédé de l'invention en milieu défini selon l'invention avec ajout d'une solution d'enrichissement en macroéléments, contenant notamment de l'azote et du phosphore, en microéléments et en vitamines et ajout d'une solution d'enrichissement de type C/N/P (Exemple 5). La Figure 5A présente l'évolution de la quantité de matière sèche produite au cours de l'essai. La Figure 5B présente le profil lipidique de l'huile contenue dans la souche CCAP 1104/3 en fin de culture.

### Exemples :

### Exemple 1 : culture d'une souche de l'espèce Crypthecodinium cohnii (souche CCAP 1104/3) selon le procédé de l'invention en milieu chimiquement défini selon l'invention avec ajout d'une solution d'enrichissement en source carbonée.

Les cultures de *Crypthecodinium cohnii* (souche CCAP 1104/3) ont été réalisées dans des fermenteurs (bioréacteurs) de 1 à 5 L utiles avec automates dédiés et supervision par station informatique. Le système a été régulé en pH via l'ajout d'acide (H₂SO₄). La température de culture a été fixée à 28°C. L'agitation a été réalisée grâce à 2 mobiles d'agitation, Rushton et HTPG4. La pression d'oxygène dissout a été régulée dans le milieu tout au long de la culture, par la vitesse d'agitation (200 - 800 t/min), le débit d'air (0,6 - 1,6 vvm), voire le débit d'oxygène (0 - 1,6 vvm). Les paramètres de régulation, intégrés dans l'automate de supervision, ont permis de maintenir une pO₂ comprise entre 25% et 35%.

La concentration en biomasse totale a été suivie par mesure de la masse sèche (filtration sur filtre GF/F, Whatman, puis séchage en étuve, à 105°C, pendant 24 h minimum avant pesée).

Les analyses en contenus lipidiques totaux et des FAMEs ont été réalisées selon les méthodes classiquement décrites dans la littérature [Folch J, et al., A simple method for the isolation and purification of total lipides from animal tissues. J Biol Chem. 1957 May; 226(1):497-509].

La culture est réalisée dans le milieu défini selon l'invention dont la composition est donnée au tableau 2 ci-dessous.

**Tableau 2**

| | Ingrédient | Concentration en g/L |
|---|---|---|
| Macroéléments | NaCl | 5 |
| | MgCl₂ 6H₂O | 1 |
| | NₐHCO₃ | 0,19 |
| | K₂HPO₄ | 0,5 |
| | CaCl₂ 2H₂O | 1,9 |
| | FeCl₃ 6H₂O | 0,02 |
| | H₃BO₃ | 0,06 |
| | KNO₃ | 2,4 |
| Microéléments | Na₂EDTA | 0,03 |
| | MnCl₂ 4H₂O | 0,0045 |
| | ZnCl₂ | 0,0003 |
| | CoCl₂ 6H₂O | 0,00015 |
| | CuSO₄5H₂O | 0,0018 |
| | NiSO₄ 6H₂O | 0,0014 |
| Vitamines | Biotine | 0,000015 |
| | Thiamine | 0,008 |
| | Cobalamine | 0,00013 |
| | Panthoténate | 0,0027 |
| Source carbonée | Glycérol | 30 |

Ce milieu chimiquement défini établit une concentration de référence "1X" pour chacun de ses composants.

Une solution d'enrichissement en source carbonée organique, composée de glycérol à 712,3 g/L, permet d'alimenter le milieu lorsque la concentration en glycérol mesurée dans le fermenteur descend en dessous du seuil de 10g/L. Cela permet de maintenir la concentration en glycérol comprise entre 10g/L et 60g/L, plus préférentiellement entre 20g/L et 50g/L.

Une culture témoin est réalisée dans un milieu riche composé de 8,7 g/L d'extrait de levure, de 0,75 g/L de KNO₃, de 5 g/L de sels marins et de 30 g/L de glycérol. Une première solution d'enrichissement pour la culture témoin se compose de KNO₃ et d'extrait de levure concentrés. Ces ajouts permettent d'augmenter la quantité initialement apportée 1X de KNO3 et d'extrait de levure jusqu'à une quantité 3X. Une solution d'enrichissement en source carbonée organique, composée de glycérol à 712,3 g/L, permet d'alimenter le milieu lorsque la concentration en glycérol mesurée dans le fermenteur descend en dessous du seuil de 10g/L. Cela permet de maintenir la concentration en glycérol comprise entre 10g/L et 60g/L, plus préférentiellement entre 20g/L et 50g/L.

La Figure 1 présente les résultats obtenus au cours de cet essai.

La Figure 1A présente l'évolution de la quantité de matière sèche produite au cours de l'essai ainsi que la quantité de matière grasse produite rapportée à la quantité de matière sèche produite (%MG/MS) en fonction du temps dans un milieu selon l'invention (Milieu minéral 1X) en comparaison d'une culture en milieu riche.

En termes de biomasse on obtient 67,4 g/L avec le milieu selon l'invention (par rapport à 174,3 g/L avec le milieu riche) après 236 h de culture, avec environ 15,9% de matière grasse (pour 13,7% avec le milieu riche).

La Figure 1B présente le profil lipidique de l'huile contenue dans la souche CCAP 1104/3 en fin de culture. On remarque notamment que les PUFAs autres que le DHA sont à l'état de trace (< 1%, préférentiellement < 0,8%).

De plus, la teneur en DHA dans la matière grasse s'élève à 30,4% contre 39,7% dans le milieu riche.

Ainsi, la croissance de la souche sur le milieu de l'invention permet d'atteindre de fortes densités cellulaires, supérieures à 50g/L de biomasse, avec l'ajout d'une solution d'enrichissement en source carbonée, mais ne permet pas d'égaler les performances obtenues sur le milieu riche témoin.

### Exemple 2 : culture d'une souche de l'espèce Crypthecodinium cohnii (souche CCAP 1104/3) selon le procédé de l'invention en milieu défini selon l'invention avec ajout d'une solution d'enrichissement de source carbonée et d'une solution d'enrichissement de source d'azote et de phosphore.

Les résultats de cet exemple sont illustrés par la figure 2.

Les cultures de *Crypthecodinium* (souche CCAP 1104/3) ont été réalisées dans des fermenteurs, de manière similaire à celle décrite dans l'Exemple 1, mais avec apport supplémentaire d'une solution d'enrichissement en minéraux composée de KNO₃ à la concentration de 64,0 g/L et de K₂HPO₄ à la concentration de 4,3 g/L qui permet d'augmenter les quantités d'azote et de phosphore initiales et permet de maintenir la productivité et ainsi de poursuivre la croissance. Les ajouts de cette solution d'enrichissement en minéraux sont réalisées dès que la culture atteint une densité cellulaire de 3.10⁷ cellules/mL, soit après 92h de culture.

Une culture témoin est réalisée dans un milieu complexe, comme décrit dans l'Exemple 1.

Dans les conditions de cet exemple, la densité cellulaire et la teneur en lipides des cellules sont proches de celles obtenues dans un milieu complexe, après 330h de culture (Figure 2A).

En termes de biomasse on obtient 168,6g/L avec le milieu selon l'invention (par rapport à 210 g/L avec le milieu riche), avec environ 10% de matière grasse.

La Figure 2B présente le profil lipidique de l'huile contenue dans la souche CCAP 1104/3 en fin de culture. On remarque notamment que les PUFAs autres que le DHA sont à l'état de trace (< 1%, préférentiellement < 0,8%). De plus, la quantité de DHA est équivalente au profil obtenu dans un milieu complexe, avec 40,4% contre 39,7% de DHA dans un milieu riche.

Ainsi, la croissance de la souche sur le milieu de l'invention, avec l'ajout d'une solution d'enrichissement en source carbonée couplé à l'ajout d'une solution d'enrichissement de source d'azote et de phosphore, permet d'atteindre de fortes densités cellulaires proches de celles obtenues sur le milieu riche témoin, et permet d'obtenir une teneur en DHA dans la matière grasse identique à celle du milieu riche témoin.

### Exemple 3 : culture d'une souche de l'espèce Crypthecodinium cohnii (souche CCAP 1104/3) selon le procédé de l'invention en milieu défini selon l'invention avec ajout d'une solution d'enrichissement en source carbonée et d'une solution d'enrichissement en macroéléments, contenant notamment de l'azote et du phosphore, en microéléments et en vitamines.

Les résultats de cet exemple sont illustrés par la figure 3.

Les cultures de *Crypthecodinium* (souche CCAP 1104/3) ont été réalisées dans des fermenteurs, de manière similaire à celle décrite dans l'Exemple 2, mais avec une modification de la solution d'enrichissement de minéraux.

Dans cet exemple, la solution d'enrichissement de minéraux se compose de l'ensemble des minéraux et vitamines présents dans le milieu de culture initial. L'ajout permet d'apporter l'équivalent de deux fois la quantité de minéraux et vitamines présents dans le milieu de culture initial, pour un apport total en minéraux et vitamines correspondant à trois fois la quantité initiale ("3X "total) après ajouts.

Une culture témoin est réalisée dans un milieu complexe, comme décrit dans l'Exemple 1.

L'apport progressif de minéraux et vitamines permet d'atteindre jusqu'à 274,6 g/L de matière sèche composée de 18% de matière grasse avec une teneur en DHA de 32,6% (voir la Figure 3A et B).

Ainsi, les conditions de l'Exemple 3 permettent de dépasser les performances obtenues sur le milieu riche et sur le milieu minéral de l'Exemple 2.

La Figure 3B montre le profil lipidique de l'huile contenue dans la souche CCAP 1104/3 en fin de culture. On remarque notamment que les PUFAs autres que le DHA sont à l'état de trace (<1 %, préférentiellement < 0,8%).

De plus, la teneur en DHA dans la matière grasse s'élève à 32,8% contre 39,7% dans le milieu riche.

Ainsi, la croissance de la souche sur le milieu de l'invention, avec l'ajout d'une solution d'enrichissement en source carbonée couplé à l'ajout d'une solution d'enrichissement en macroéléments, contenant notamment de l'azote et du phosphore, en microéléments et en vitamines, permet d'atteindre de fortes densités cellulaires supérieures à celles obtenues sur le milieu riche témoin, et permet d'obtenir une teneur en DHA dans la matière grasse proche de celle du milieu riche témoin.

### Exemple 4 : culture d'une souche de l'espèce Crypthecodinium cohnii (souche CCAP 1104/3) selon le procédé de l'invention en milieu défini selon l'invention avec ajout d'une solution d'enrichissement de type C/N/P

Les résultats de cet exemple sont illustrés par la figure 4.

Les cultures de *Crypthecodinium* (souche CCAP 1104/3) ont été réalisées dans des fermenteurs, de manière similaire à celle décrite dans l'Exemple 2.

La modification de la procédure a été réalisée sur la nature des solutions d'enrichissement. Dans cet exemple, le substrat additionnel est ajouté sous forme d'ajouts d'une solution dite "solution nutritive C/N/P". La solution nutritive est composée d'un substrat carboné (glycérol), d'une source d'azote minéral (KNO₃) et d'une source de phosphore minéral (K₂HPO₄). Cette solution nutritive présente un ratio molaire en atomes de carbone, d'azote et de phosphore défini : 891 :26 :1. Ce ratio a été ajusté par les inventeurs afin de créer une limitation définie en azote et phosphore, et ainsi orienter préférentiellement le métabolisme vers la production de DHA.

Une culture témoin est réalisée dans un milieu riche comme décrit dans l'Exemple 1.

L'ajout d'une solution de C/N/P à un ratio molaire de 891:26:1 permet d'atteindre 251,2 g/L de matière sèche composée de 8% de matière grasse avec une teneur en DHA de 59,3% (Figures 4A et B).

Ainsi, selon ce mode de réalisation de l'invention, l'apport en sources de carbone, d'azote et de phosphore durant la culture dans un milieu chimiquement défini, permet d'augmenter la teneur en DHA dans la matière grasse jusqu'à la valeur de 59,3%. Cette performance dépasse toutes celles décrites dans l'art antérieur, que ce soit dans un milieu minéral ou dans un milieu riche. Cette performance permet également d'améliorer les procédés décrits dans les Exemples 1, 2 et 3.

La Figure 4B montre le profil lipidique de l'huile contenue dans la souche CCAP 1104/3 en fin de culture. On remarque notamment que les PUFAs autres que le DHA sont à l'état de trace (<1%, préférentiellement < 0,8%).

De plus, la teneur en DHA dans la matière grasse s'élève à 59,3%, contre 39,7% de DHA dans un milieu riche.

Ainsi, en comparaison avec les Exemples 1, 2 et 3, l'apport contrôlé de source de carbone, d'azote et de phosphore (solution d'enrichissement de type C/N/P selon le ratio molaire 891 :26 :1) permet d'enrichir significativement l'huile en DHA, tout en maintenant des densités et des productivités élevées.

### Conclusions à la lecture des résultats des exemples

Les souches de microalgues visées par l'invention cultivées selon l'invention permettent de produire des quantités significatives de biomasse, contenant des lipides riches en DHA et pauvres en DPA.

En effet, le procédé de l'invention en conditions hétérotrophe ou mixotrophe permet d'obtenir une concentration en biomasse au moins égale sinon supérieure à 50 g/L de matière sèche et pouvant atteindre jusqu'à plus de 250 g/L de matière sèche. Cette matière sèche peut être composée de plus de 10% de matière grasse, cette teneur pouvant aller potentiellement jusqu'à 60% de lipides (Figure 3A) avec une teneur en DHA pouvant aller jusqu'à plus de 59% (Figure 4B).

De manière générale, la quantité de DHA obtenue avec le procédé de l'invention est au moins aussi importante que celle obtenue en utilisant des milieux de culture riches tels que décrits dans l'art antérieur. Par exemple, les résultats de l'Exemple 2 montrent que l'on obtient 40,4% de DHA avec la culture en milieu défini selon l'invention (tableau 1) en bioréacteur et on obtient 39,7% de DHA pour la culture témoin (milieu complexe).

Les autres PUFAs non souhaités, en particulier, le DPA, représentent en général moins de 3%, et jusqu'à moins de 0,01% des acides gras.

De manière générale selon le procédé de l'invention, les PUFAs autres que le DHA sont à l'état de trace (< 1%, préférentiellement < 0,8%).

### Exemple 5 : culture d'une souche de l'espèce Crypthecodinium cohnii (souche CCAP 1104/3) selon le procédé de l'invention en milieu défini selon l'invention avec ajout d'une solution d'enrichissement en macroéléments, contenant notamment de l'azote et du phosphore, en microéléments et en vitamines et ajout d'une solution d'enrichissement de type C/N/P

La culture a été réalisée dans des conditions similaires à celle de l'exemple 1.

La culture est réalisée dans le milieu défini selon l'invention dont la composition est donnée au tableau 3 ci-dessous.

**Tableau 3**

| | Composé | Concentration (g/L) |
|---|---|---|
| Solution Macro-éléments | NaCl | 4 |
| | MgCl₂ 6H₂O | 0,97 |
| | NaHCO₃ | 0,19 |
| | KNO₃ | 3,69 |
| | K₂HPO₄ | 0,5 |
| | CaCL₂ 2H₂O | 1,9 |
| | FeCl₃ 6H₂O | 0,02 |
| | H₃BO₃ | 0,06 |
| Solution Micro-éléments | Na₂EDTA | 0 |
| | MnCL₂ 4H₂O | 0,009 |
| | ZnCL₂ | 0,0006 |
| | CoCL₂ 6H₂O | 0,0003 |
| | CuSO₄ 5H₂O | 0,000034 |
| | NiSO₄ 6H₂0 | 0,00033 |
| Solution Vitamines | Biotine | 0,0000075 |
| | Thiamine | 0,004 |
| | Vitamine B12 | 0,00006 |
| | Panthoténate | 0,0014 |
| Source Carbonée | Glycérol | 20 |

Dans cet exemple, une solution d'enrichissement de minéraux composée de l'ensemble des minéraux et vitamines présents dans le milieu de culture initial à l'exclusion du KNO₃ et du KH₂PO₄ est utilisée pour enrichir le milieu pour un apport total en minéraux et vitamines correspondant à trois fois la quantité initiale ("3X " total) après ajouts. De plus le substrat additionnel est ajouté sous forme d'ajouts d'une solution dite "solution nutritive C/N/P". La solution nutritive est composée d'un substrat carboné (glycérol), d'une source d'azote minéral (KN03) et d'une source de phosphore minéral (K2HPO4). Cette solution nutritive présente un ratio molaire en atomes de carbone, d'azote et de phosphore défini : 445/13/1. Ce ratio a été ajusté afin de créer une limitation définie en azote et phosphore, et ainsi orienter préférentiellement le métabolisme vers la production de DHA tout en maintenant la biomasse dans un état permettant une activité catalytique suffisante pour maintenir une bonne productivité.

Une culture témoin est réalisée dans un milieu riche comme décrit dans l'Exemple 1.

L'ajout de la solution d'enrichissement en minéraux et vitamines et de la solution de C/N/P à un ratio molaire de 445/13/1 permet d'atteindre 275 g/L de matière sèche composée de 18% de matière grasse avec une teneur en DHA de 37% (Figures 5A et B).

Ainsi, selon ce mode de réalisation de l'invention, l'apport en minéraux et vitamines d'une part et en sources de carbone, d'azote et de phosphore d'autre part, durant la culture dans un milieu chimiquement défini, permet d'obtenir des performances en terme d'accumulation en acides gras et teneur en DHA satisfaisantes tout en obtenant une bonne productivité.

La Figure 5B montre le profil lipidique de l'huile contenue dans la souche CCAP 1104/3 en fin de culture. On remarque notamment que les PUFAs autres que le DHA sont à l'état de trace (<1%).

## Revendications

1. Procédé de culture de souches de microalgues du genre *Crypthecodinium* présentant un gène codant la petite sous-unité de l'ARN ribosomique 18s présentant une identité génétique d'au moins 96% avec le même gène de la souche de l'espèce *Crypthecodinium cohnii,* déposée à la CCAP (Culture Collection of Algae and Protozoa), sous le numéro CCAP 1104/3 pour la préparation d'une biomasse d'une densité au moins supérieure à 50 g/L de matière sèche, présentant un taux de DHA au moins supérieur à 5 g/L dans la culture, **caractérisé en ce que** qu'il comprend au moins une première étape dans laquelle on cultive lesdites souches dans un milieu chimiquement défini pendant un temps suffisant pour atteindre une concentration en souches comprise entre 5.10⁶ et 5.10⁸ cellules/mL, ledit milieu chimiquement défini comprenant
- à titre de macroéléments du chlorure de sodium (NaCl), du chlorure de magnésium, du bicarbonate de sodium (NₐHCO₃), de l'hydrogénophosphate de potassium (K₂HPO₄), du chlorure de calcium, du chlorure de fer (III) (FeCl₃ 6H₂O), de l'acide borique (H₃BO₃) et une source d'azote
- à titre de microéléments de l'acide éthylène diamine tétraacétique sous forme disodique (Na₂EDTA), du chlorure de manganèse (II) tetrahydrate (MnCl₂ 4H₂O), du chlorure de zinc (ZnCl₂), du chlorure de cobalt hexahydrate (CoCl₂ 6H₂O), du sulfate de cuivre pentahydrate (CuSO₄ 5H₂O), et du sulfate de nickel hexahydrate (NiSO₄ 6H₂O) ;
- à titre de vitamine de la biotine, de la thiamine, de la cobalamine ou vitamine B12 et du panthoténate ou vitamine B5,
- et une source carbonée,
et au moins une deuxième étape dans laquelle on ajoute au milieu de culture, simultanément ou successivement, une ou plusieurs solution(s) d'enrichissement en source carbonée comprenant à la fois la source de carbone, la source d'azote et la source de phosphore (solution d'enrichissement C/N/P).

2. Procédé selon la revendication 1, **caractérisé en ce que** dans le milieu chimiquement défini le chlorure de sodium est à une concentration comprise entre 2 et 8 g/L, le chlorure de magnésium est à une concentration comprise entre 0,6 et 1,4 g/L, le bicarbonate de sodium est à une concentration comprise entre 0,14 et 0,26 g/L, la source d'azote est à une concentration comprise entre 1,7 et 3,1 g/L, l'hydrogénophosphate de potassium est à une concentration comprise entre 0,3 et 0,7 g/L, le chlorure de calcium est à une concentration comprise entre 1,4 et 2,6 g/L, le chlorure de fer (III) est à une concentration comprise entre 0,016 et 0,024 g/L, l'acide borique est à une concentration comprise entre 0,046 et 0,74 g/L, l'acide éthylène diamine tétraacétique sous forme disodique est à une concentration comprise entre 0 et 0,08 g/L, le chlorure de manganèse (II) tetrahydrate est à une concentration comprise entre 0,001 et 0,02 g/L, le chlorure de zinc est à une concentration comprise entre 0,0001 et 0,0008 g/L, le chlorure de cobalt hexahydrate est à une concentration comprise entre 0,00005 et 0,0005 g/L, le sulfate de cuivre pentahydrate est à une concentration comprise entre 0,00007 et 0,004 g/L, le sulfate de nickel hexahydrate est à une concentration comprise entre 0,0005 et 0,003 g/L, la biotine est à une concentration comprise entre 0,5.16⁻⁶ et 1,5.10⁻⁴ g/L, la thiamine est à une concentration comprise entre 0,5.16⁻⁶ et 1,5.10⁻⁴ g/L, la cobalamine est à une concentration comprise entre 0,5.16⁻⁶ et 1,5.10⁻⁴ g/L et le panthoténate est à une concentration comprise entre 0,5.16⁻⁶ et 1,5.10⁻⁴ g/L.

3. Procédé selon l'une quelconque des revendications 1 ou 2, **caractérisé en ce que** la source d'azote est un sel de nitrate ou un sel de glutamate ou un sel d'ammonium ou de l'urée.

4. Procédé selon l'une quelconque des revendications 1 à 3, **caractérisé en ce que** la source carbonée est un (ou des) glucide(s), un (ou des) acétate(s), un ou des alcools, une ou des molécules complexes ou leur mélange.

5. Procédé selon l'une quelconque des revendications 1 à 4, **caractérisé en ce qu'**à la première étape la source carbonée est à une concentration comprise entre 10 mM et 500 mM.

6. Procédé selon l'une quelconque des revendications 1 à 5, **caractérisé en ce que** la culture est en hétérotrophie ou en mixotrophie.

7. Procédé selon l'une quelconque des revendications 1 à 6, **caractérisé en ce que** la solution d'enrichissement C/N/P à quantité de carbone fixe jusqu'à deux fois plus ou deux fois moins d'azote, et/ou jusqu'à deux fois plus ou deux fois moins de phosphore par rapport au ratio 891 :26 :1.

8. Procédé selon la revendication 7, **caractérisé en ce que** la solution d'enrichissement C/N/P présente les ratios molaires choisi parmi : 891 :52 :1, 891:26:1 et 891 :13 :1, 445 :26 :1, 445 :13 :1 et 445 :6,5 :1, 1782 :104 :1, 1782 :52 :1, et 1782 :26 : 1.

9. Procédé selon l'une des revendications 1 à 8, **caractérisé en ce qu'**il comprend une étape de récupération de la biomasse.

10. Procédé selon la revendication 9, **caractérisé en ce que** la biomasse dans la culture présente une densité au moins supérieure à 100 g/L en matière sèche.

11. Procédé selon l'une des revendications 9 ou 10, **caractérisée en ce que** la biomasse présente un taux de DHA au moins supérieur à 10 g/L dans la culture.

12. Procédé selon l'une quelconque des revendications 9 à 11, **caractérisée en ce que** la biomasse présente un taux de PUFAs autres que le DHA inférieur à 1 % de la teneur totale en acide gras.

13. Procédé de préparation de DHA **caractérisé en ce qu'**il comprend les étapes de culture et de récupération de la biomasse selon l'une des revendications 9 à 12, d'extraction des lipides à partir de ladite biomasse, et d'extraction du DHA des lipides récupérés.

14. Procédé selon la revendication 13 **caractérisé en ce qu'**il comprend en outre une étape de purification du DHA produit sous forme d'esters de DHA.

## Patentansprüche

1. Verfahren zur Züchtung von Mikroalgenstämmen der Gattung *Crypthecodinium,* aufweisend ein Gen, das die kleine Untereinheit der Ribosomen-RNA 18s codiert, aufweisend eine genetische Identität von mindestens 96% mit demselben Gen des Stamms der Art *Crypthecodinium cohnii,* hinterlegt bei der CCAP (Culture Collection of Algae and Protozoa) unter der Nummer CCAP 1104/3, für die Herstellung einer Biomasse mit einer Dichte mindestens über 50 g/L Trockenmasse, aufweisend einen DHA-Gehalt mindestens über 5 g/L in der Kultur, **dadurch gekennzeichnet, dass** es mindestens einen ersten Schritt umfasst, bei dem die Stämme in einem chemisch definierten Milieu während einer Zeit kultiviert werden, die ausreichend ist, um eine Stammkonzentration zwischen 5.10⁶ und 5.10⁸ Zellen/mL zu erreichen, wobei das chemisch definierte Milieu umfasst
- als Makroelemente Natriumchlorid (NaCl), Magnesiumchlorid, Natriumbicarbonat (NaHCO₃), Kaliumhydrogenphosphat (K₂HPO₄), Calciumchlorid, Eisen(III)-chlorid (FeCl₃ 6H₂O), Borsäure (H₃BO₃) und eine Stickstoffquelle
- als Mikroelemente Ethylendiamintetraessigsäure in disodischer Form (Na₂EDTA), Mangan(II)-chlorid-Tetrahydrat (MnCl₂ 4H₂O), Zinkchlorid (ZnCl₂), Cobalt(II)-chlorid-Hexahydrat (CoCl₂ 6H₂O), Kupfersulfat-Pentahydrat (CuSO₄ 5H₂O) und Nickelsulfat-Hexahydrat (NiSO₄ 6H₂O);
- als Vitamin Biotin, Thiamin, Cobalamin oder Vitamin B12 und Panthotenat oder Vitamin B5,
- und einen kohlenstoffhaltigen Stamm,
und mindestens einen zweiten Schritt, bei dem dem Kulturmilieu gleichzeitig oder nacheinander eine oder mehrere Anreichungslösung(en) mit kohlenstoffhaltigem Stamm hinzugefügt werden, umfassend sowohl die Kohlenstoffquelle, die Stickstoffquelle und die Phosphorquelle (Anreichungslösung C/N/P).

2. Verfahren nach Anspruch 1, **dadurch gekennzeichnet, dass** in dem chemisch definierten Milieu das Natriumchlorid in einer Konzentration zwischen 2 und 8 g/L vorliegt, das Magnesiumchlorid in einer Konzentration zwischen 0,6 und 1,4 g/L vorliegt, das Natriumbicarbonat in einer Konzentration zwischen 0,14 und 0,26 g/L vorliegt, die Stickstoffquelle in einer Konzentration zwischen 1,7 und 3,1 g/L vorliegt, das Kaliumhydrogenphosphat in einer Konzentration zwischen 0,3 und 0,7 g/L vorliegt, das Calciumchlorid in einer Konzentration zwischen 1,4 und 2,6 g/L vorliegt, das Eisen(III)-chlorid in einer Konzentration zwischen 0,016 und 0,024 g/L vorliegt, die Borsäure in einer Konzentration zwischen 0,046 und 0,74 g/L vorliegt, die Ethylendiamintetraessigsäure in disodischer Form in einer Konzentration zwischen 0 und 0,08 g/L vorliegt, das Mangan(II)-chlorid-Tetrahydrat in einer Konzentration zwischen 0,001 und 0,02 g/L vorliegt, das Zinkchlorid in einer Konzentration zwischen 0,0001 und 0,0008 g/L vorliegt, das Cobalt(II)-chlorid-Hexahydrat in einer Konzentration zwischen 0,00005 und 0,0005 g/L vorliegt, das Kupfersulfat-Pentahydrat in einer Konzentration zwischen 0,00007 und 0,004 g/L vorliegt, das Nickelsulfat-Hexahydrat in einer Konzentration zwischen 0,0005 und 0,003 g/L vorliegt, das Biotin in einer Konzentration zwischen 0,5.16⁻⁶ und 1,5.10⁻⁴ g/L vorliegt, das Thiamin in einer Konzentration zwischen 0,5.16⁻⁶ und 1,5.10⁻⁴ g/L vorliegt, das Cobalamin in einer Konzentration zwischen 0,5.16⁻⁶ und 1,5.10⁻⁴ g/L vorliegt und das Panthotenat in einer Konzentration zwischen 0,5.16⁻⁶ und 1,5.10⁻⁴ g/L vorliegt.

3. Verfahren nach einem der Ansprüche 1 oder 2, **dadurch gekennzeichnet, dass** die Stickstoffquelle ein Nitratsalz oder ein Glutamatsalz oder ein Ammoniumsalz oder Harnstoff ist.

4. Verfahren nach einem der Ansprüche 1 bis 3, **dadurch gekennzeichnet, dass** die kohlenstoffhaltige Quelle ein (oder) Glucid(e), ein (oder) Acetat(e), ein (oder) Alkohol(e), ein oder komplexe Moleküle oder ihr Gemisch ist.

5. Verfahren nach einem der Ansprüche 1 bis 4, **dadurch gekennzeichnet, dass** beim ersten Schritt die kohlenstoffhaltige Quelle in einer Konzentration zwischen 10 mM und 500 mM vorliegt.

6. Verfahren nach einem der Ansprüche 1 bis 5, **dadurch gekennzeichnet, dass** die Kultur heterotroph oder mixotroph ist.

7. Verfahren nach einem der Ansprüche 1 bis 6, **dadurch gekennzeichnet, dass** die Kohlenstoffmengen-Anreicherungslösung C/N/P bis zu zweimal mehr oder zweimal weniger Stickstoff und/oder bis zu zweimal mehr oder zweimal weniger Phosphor bindet in Bezug auf das Verhältnis 891:26:1.

8. Verfahren nach Anspruch 7, **dadurch gekennzeichnet, dass** die Anreicherungslösung C/N/P die molaren Verhältnisse aufweist, die aus 891:52:1, 891:26:1 und 891:13:1, 445:26:1, 445:13:1 und 445:6,5:1, 1782:104:1, 1782:52:1, und 1782:26:1 ausgewählt sind.

9. Verfahren nach einem der Ansprüche 1 bis 8, **dadurch gekennzeichnet, dass** es einen Schritt der Rückgewinnung der Biomasse umfasst.

10. Verfahren nach Anspruch 9, **dadurch gekennzeichnet, dass** die Biomasse in der Kultur eine Dichte mindestens über 100 g/L Trockenmasse aufweist.

11. Verfahren nach einem der Ansprüche 9 oder 10, **dadurch gekennzeichnet, dass** die Biomasse einen DHA-Gehalt mindestens über 10 g/L in der Kultur aufweist.

12. Verfahren nach einem der Ansprüche 9 bis 11, **dadurch gekennzeichnet, dass** die Biomasse einen Gehalt an PUFAs, die keine DHA sind, unter 1% des Gesamtanteils an Fettsäuren aufweist.

13. Verfahren zur Herstellung von DHA, **dadurch gekennzeichnet, dass** es die Schritte des Kultivierens und des Rückgewinnens der Biomasse nach einem der Ansprüche 9 bis 12, der Extraktion der Lipide aus der Biomasse und der Extraktion der DHA aus den rückgewonnenen Lipiden umfasst.

14. Verfahren nach Anspruch 13, **dadurch gekennzeichnet, dass** es ferner einen Reinigungsschritt der DHA umfasst, hergestellt in Form von DHA-Estern.

## Claims

1. Process for culturing microalgae strains of the genus *Crypthecodinium* having a gene encoding the small subunit 18s ribosomal RNA having a genetic similarity of at least 96% with the same gene of the strain of the species *Crypthecodinium cohnii,* deposited in the CCAP (Culture Collection of Algae and Protozoa) under number CCAP 1104/3, for the production of biomass with a density at least greater than 50 g/L dry matter, having a DHA content at least greater than 5 g/L in the culture, **characterized in that** it comprises at least a first step wherein said strains are cultivated in a chemically defined medium for a sufficient time to achieve a strain concentration between 5·10⁶ and 5·10⁸ cells/mL, said chemically defined medium comprising
- as macroelements: sodium chloride (NaCl), magnesium chloride, sodium bicarbonate (NaHCO₃), potassium hydrogen phosphate (K₂HPO₄), calcium chloride, iron (III) chloride (FeCl₃ 6H₂O), boric acid (H₃BO₃) and a nitrogen source
- as microelements: disodium ethylenediaminetetraacetic acid (Na₂EDTA), manganese (II) chloride tetrahydrate (MnCl₂ 4H₂O), zinc chloride (ZnCl₂), cobalt chloride hexahydrate (CoCl₂ 6H₂O), copper sulfate pentahydrate (CuSO₄ 5H₂O), and nickel sulfate hexahydrate (NiSO₄ 6H₂O);
- as vitamin: biotin, thiamine, cobalamin or vitamin B12 and pantothenate or vitamin B5,
- and a carbon source,
and at least a second step wherein one or more carbon source enrichment solution(s) comprising at the same time the carbon source, the nitrogen source and the phosphorus source (C/N/P enrichment solution) are added, simultaneously or successively, to the culture medium.

2. Process according to claim 1, **characterized in that** in the chemically defined medium sodium chloride is at a concentration between 2 and 8 g/L, magnesium chloride is at a concentration between 0.6 and 1.4 g/L, sodium bicarbonate is at a concentration between 0.14 and 0.26 g/L, the nitrogen source is at a concentration between 1.7 and 3.1 g/L, potassium hydrogen phosphate is at a concentration between 0.3 and 0.7 g/L, calcium chloride is at a concentration between 1.4 and 2.6 g/L, iron (III) chloride is at a concentration between 0.016 and 0.024 g/L, boric acid is at a concentration between 0.046 and 0.74 g/L, disodium ethylenediaminetetraacetic acid is at a concentration between 0 and 0.08 g/L, manganese (II) chloride tetrahydrate is at a concentration between 0.001 and 0.02 g/L, zinc chloride is at a concentration between 0.0001 and 0.0008 g/L, cobalt chloride hexahydrate is at a concentration between 0.00005 and 0.0005 g/L, copper sulfate pentahydrate is at a concentration between 0.00007 and 0.004 g/L, nickel sulfate hexahydrate is at a concentration between 0.0005 and 0.003 g/L, biotin is at a concentration between 0.5·16⁻⁶ and 1.5·10⁻⁴ g/L, thiamine is at a concentration between 0.5·16⁻⁶ and 1.5·10⁻⁴ g/L, cobalamin is at a concentration between 0.5·16⁻⁶ and 1.5·10⁻⁴ g/L and pantothenate is at a concentration between 0.5·16⁻⁶ and 1.5·10⁻⁴ g/L.

3. Process according to claim 1 or 2, **characterized in that** the nitrogen source is a nitrate salt or a glutamate salt or an ammonium salt or urea.

4. Process according to any one of claims 1 to 3, **characterized in that** the carbon source is one (or more) carbohydrate(s), one (or more) acetate(s), one or more alcohols, one or more complex molecules or mixture thereof.

5. Process according to any one of claims 1 to 4, **characterized in that** in the first step the carbon source is at a concentration between 10 mM and 500 mM.

6. Process according to any one of claims 1 to 5, **characterized in that** the culture is heterotrophic or mixotrophic.

7. Process according to any one of claims 1 to 6, **characterized in that** the C/N/P enrichment solution has for a fixed quantity of carbon up to two times more or two times less nitrogen, and/or up to two times more or two times less phosphorus in relation to the ratio 891:26:1.

8. Process according to claim 7, **characterized in that** the C/N/P enrichment solution has the molar ratios selected from: 891:52:1, 891:26:1 and 891:13:1, 445:26:1, 445:13:1 and 445:6.5:1, 1782:104:1, 1782:52:1, and 1782:26:1.

9. Process according to one of claims 1 to 8, **characterized in that** it comprises a biomass harvesting step.

10. Process according to claim 9, **characterized in that** the biomass is cultured at a density at least greater than 100 g/L dry matter.

11. Process according to claim 9 or 10, **characterized in that** the biomass has a DHA content at least greater than 10 g/L in the culture.

12. Process according to any one of claims 9 to 11, **characterized in that** the biomass has a level of PUFAs other than DHA lower than 1% of the total fatty acid content.

13. Process for producing DHA, **characterized in that** it comprises the steps of culturing and harvesting the biomass according to one of claims 9 to 12, extracting lipids from said biomass, and extracting DHA from the recovered lipids.

14. Process according to claim 13, **characterized in that** it further comprises a step of purifying the DHA produced in the form of DHA esters.
